# EUROPEAN PATENT SPECIFICATION

(11) **EP 0 291 587 B1**
(45) Date of publication and mention of the grant of the patent: **27.07.1994**
(21) Application number: 87304365.7
(22) Date of filing: 18.05.1987
(51) Int. Cl.: A61L 15/16

(54) **Wound dressing**
Wundverband
Pansement pour blessure

(43) Date of publication of application: 23.11.1988
(60) Divisional application: 91201911.4
(73) Proprietor: BRITISH TEXTILE TECHNOLOGY GROUP, Didsbury Manchester M20 8RX (GB)
(72) Inventor: Sagar, Brian, Cheadle Cheshire (GB); Hamlyn, Paul, East Didsbury Manchester (GB); Wales, David, Reddish Stockport Cheshire (GB)
(74) Representative: Lawrence, John Gordon

(56) References cited:
- GB-A- 2 148 959
- GB-A- 2 182 560

## Description

This invention concerns a wound dressing.

The wound healing properties of chitin and chitin derivatives have long been recognised and documented. Present practice suggests that wounds should be kept moist to prevent scabbing, reduce the development of scar tissue and minimise healing time.

The extraction of chitin from its natural sources and its incorporation in conventional wound dressings is quite costly.

GB-A-2,148,959 discloses the production of non-woven fabric comprised by a wet-laid mat of microfungal hyphae treated with a plasticiser before the hyphae are allowed to dry to prevent embrittlement. Among application envisaged for such fabrics are mentioned wound dressings. However, for a wound dressing GB-A-2,148,959 prescribes laminating the mat, after perforating, as by needle punching, with an absorbent backing of conventional textile material.

The present invention avoids the need for any such lamination to conventional textile material and provides an improved wound dressing adapted to current medical practice and at economic cost.

According to the present invention there is provided a wound dressing comprised by a wet-laid mat of microfungal hyphae which have been treated with alkali to expose chitin and chitosan and incorporating a plasticiser, cut to size and sealed in a watervapour-impermeable pack without being allowed to dry.

The microfungal hyphae may be vegetative hyphae or sporangiophores.

The microfungal hyphae may be Mucor mucedo or Rhizomucor miehei.

The plasticiser may be water or may be glycerol or polyethylene glycol.

The wet-laid mat or the mycelia from which it is formed may be bleached.

The wet-laid mat may incorporate other fibres of substances known to assist or facilitate wound healing, such as of collagen, a well-known haemostatic agent or of an alginate, useful as a physical barrier to prevent drying and adhesion between the wound and the dressing material. The wet-laid mat may also incorporate bound metallic silver, useful as an anti-bacterial agent.

The wound dressings may be treated with a bi-functional cross-linking agent such as glutaraldehyde to improve their strength.

The invention will be further apparent from the following description which concerns by way of example only the preparation of various forms of wound dressing embodying same and with reference to the accompanying drawings, in which:

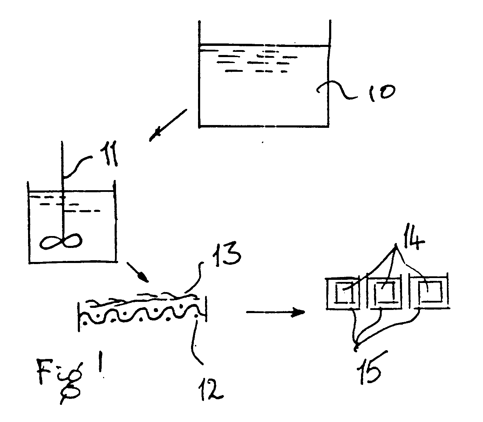

- Figure 1: is a diagrammatic illustration of a batch process for preparing a first form of wound dressing;
Referring firstly to Figure 1, micro-fungal mycelia are produced from a culture of Mucor mucedo (CMI 184 726), grown in a nutrient solution containing malt extract (17g/l) and mycological peptone (3g/l) in a fermenter vessel 10 at a temperature of 30°C for one to two days.

The culture is then washed and treated with a 2N boiling solution of sodium hydroxide for one hour to dissolve protein from the outer layers of the cell walls and expose the underlying chitin and chitosan. Further de-acetylisation of the chitin may be effected by 40% sodium hydroxide solution.

The culture is repeatedly washed until neutral pH is obtained and then bleached by treatment with a solution of hydrogen peroxide (80 ml/l 37% H₂O₂ + 40 g/l NaOH + 40 g/l sodium silicate) for two hours at room temperature.

The culture is washed again and disintegrated using normal paper making equipment 11 to ensure an even dispersion of the hyphae in water to form a slurry. The slurry is strained through a filter medium 12 to leave a wet-laid matt 13 having a thickness of 1mm or thereabouts.

If desired other fibres having wound healing properties such as of collagen or an alginate or both may be mixed with the hyphae before the matt is laid, as may textile fibres to give mechanical strength or other properties.

Suitably sized portions 14 for wound dressings of desired size are cut from the matt 13 and immediately encapsulated whilst still wet in airtight packs 15 and subsequently sterilised. The retained water acts as a plasticiser to prevent the hyphae from becoming dry and brittle and also ensures that the dressings are moist when removed from the packs for use.

Alternatively glycerol or polyethylene glycol may be added to the slurry before the matt is laid.

In another example Mucor mucedo is replaced by Rhizomucor miehei (CMI 147 066) which is fermented at 50°C.

Thus for example, the matts or pads or fibres from which they are formed may be treated in a solution of silver nitrate whereby silver ions will be captured by the chitosan and thus be present in the dressings as an anti-bacterial agent.

Again for example, the dressings may be treated with a bi-functional cross-linking agent such as glutaraldehyde.

Yet again, for example the wet-laid matts may be laminated with one or more backing layers of conventional, textile fibre if desired.

Tests carried out by Royal National Orthopaedic Hospital at Stamore Middlesex, indicated that wound dressings prepared in accordance with the invention from Mucor mucedo gave encouraging results in terms of the quality and quantity of repair tissue.

## Claims

1. A wound dressing comprised by a wet-laid mat of microfungal hyphae which have been treated with alkali to expose chitin and chitosan and incorporating a plasticiser, cut to size and sealed in a watervapour-impermeable pack without being allowed to dry.

2. A wound dressing according to claim 1, wherein the plasticiser is water.

3. A wound dressing according to claim 1, wherein the plasticiser is glycerol.

4. A wound dressing according to claim 1, wherein the plasticiser is polyethylene glycol.

5. A wound dressing according to claim 1, wherein the microfungal hyphae are Mucor mucedo.

6. A wound dressing according to claim 1, wherein the microfungal hyphae are Rhizomucor michei.

7. A wound dressing according to any preceding claim, wherein other fibres are included with the micro-fungal hyphae.

8. A wound dressing according to claim 7, wherein said other fibres comprise collagen.

9. A wound dressing according to claim 7, wherein said other fibres comprise alginate.

10. A wound dressing according to any preceding claim, wherein the alkali treated hyphae are treated with a silver salt whereby silver ions are captured and present in the dressing as an anti-bacterial agent.

11. A wound dressing according to any preceding claim, wherein the wet-laid mat is treated with a bi-functional cross-linking agent.

12. A wound dressing according to claim 11, wherein the bi-functional cross-linking agent is glutaraldehyde.

## Patentansprüche

1. Wundverband, der eine naß abgesetzte Matte aus mikropilzartigen Hyphen aufweist, die mit Alkali zum Freilegen von Chitin und Chitosan behandelt wurden, und die einen Weichmacher enthält, auf ihre Größe geschnitten und in einer wasserdampfundurchlässigen Packung ohne die Möglichkeit zu trocknen versiegelt ist.

2. Wundverband nach Anspruch 1, worin der Weichmacher Wasser ist.

3. Wundverband nach Anspruch 1, worin der Weichmacher Glyzerin ist.

4. Wundverband nach Anspruch 1, worin der Weichmacher Polyäthylenglykol ist.

5. Wundverband nach Anspruch 1, worin die pilzartigen Hyphen Mucor mucedo sind.

6. Wundverband nach Anspruch 1, worin die mikropilzartigen Hyphen Rhizomucor miehei sind.

7. Wundverband nach einem der vorhergehenden Ansprüche, worin andere Fasern mit den mikropilzartigen Hyphen enthalten sind.

8. Wundverband nach Anspruch 7, worin die anderen Fasern Kollagen aufweisen.

9. Wundverband nach Anspruch 7, worin die anderen Fasern Alginat aufweisen.

10. Wundverband nach einem der vorhergehenden Ansprüche, worin die alkalibehandelten Hyphen mit einem Silbersalz behandelt werden, wodurch Silberionen eingefangen und im Verband als antibakterielles Mittel vorhanden sind.

11. Wundverband nach einem der vorhergehenden Ansprüche, worin die naß abgesetzte Matte mit einem bifunktionellen vernetzenden Mittel behandelt ist.

12. Wundverband nach Anspruch 11, worin das bifunktionelle vernetzende Mittel Glutaraldehyd ist.

## Revendications

1. Pansement pour plaies constitué par un matelas, déposé par voie humide, d'hyphes microfongiques ayant été traitées par une base pour libérer la chitine et le chitosan et par l'incorporation d'un plastifiant, découpé à la taille voulue et enfermé dans un sachet imperméable à la vapeur d'eau sans être séché.

2. Pansement pour plaies selon la revendication 1, dans lequel le plastifiant est l'eau.

3. Pansement pour plaies selon la revendication 1, dans lequel le plastifiant est le glycérol.

4. Pansement pour plaies selon la revendication 1, dans lequel le plastifiant est le polyéthylèneglycol.

5. Pansement pour plaies selon la revendication 1, dans lequel les hyphes microfongiques sont Mucor mucedo.

6. Pansement pour plaies selon la revendication 1, dans lequel les hyphes microfongiques sont Rhizomucor michei.

7. Pansement pour plaies selon une quelconque des revendications qui précèdent, dans lequel d'autres fibres sont incluses avec les hyphes microfongiques.

8. Pansement pour plaies selon la revendication 7, dans lequel lesdites autres fibres comprennent du collagène.

9. Pansement pour plaies selon la revendication 7, dans lequel lesdites autres libres comprennent un alginate.

10. Pansement pour plaies selon une quelconque des revendications qui précèdent, dans lequel les hyphes traitées par une base sont traitées par un sel d'argent, les ions argent étant piégés et présents dans le pansement en tant qu'agent antibactérien.

11. Pansement pour plaies selon une quelconque des revendications qui précèdent, dans lequel le matelas déposé par voie humide est traité par un agent réticulant bifonctionnel.

12. Pansement pour plaies selon la revendication 11, dans lequel l'agent de réticulation bifonctionnel est le glutaraldéhyde.
